# EUROPÄISCHE PATENTSCHRIFT

(11) **EP 3 962 582 B1**
(45) Veröffentlichungstag und Bekanntmachung des Hinweises auf die Patenterteilung: **16.08.2023**
(21) Anmeldenummer: 20724762.8
(22) Anmeldetag: 30.04.2020
(51) Int. Cl.: A61N 1/05, A61L 31/00, A61B 5/00

(54) **IMPLANTIERBARE ELEKTRISCHE KONTAKTANORDNUNG**
IMPLANTABLE ELECTRICAL CONTACT ARRANGEMENT
DISPOSITIF DE CONTACT ÉLECTRIQUE IMPLANTABLE

(30) Priorität: 03.05.2019 DE 102019206388
(43) Veröffentlichungstag der Anmeldung: 09.03.2022
(73) Patentinhaber: Neuroloop GmbH, 79110 Freiburg (DE)
(72) Erfinder: KIMMIG, Fabian, 79331 Teningen (DE); BORETIUS, Tim, 79098 Freiburg (DE)
(74) Vertreter: Rösler, Uwe
(86) Internationale Anmeldenummer: PCT/EP2020/062018
(87) Internationale Veröffentlichungsnummer: WO 2020/225090

(56) Entgegenhaltungen:
- WO-A1-2017/181027
- US-A1- 2011 122 486
- US-A1- 2017 087 350

## Beschreibung

### Technisches Gebiet

Die Erfindung bezieht sich auf eine implantierbare elektrische Kontaktanordnung, die wenigstens eine Elektrodenkörperanordnung aufweist, die ansonsten vollständig in ein aus biokompatiblem, elektrisch isolierendem Material gefertigtes Trägersubstrat integriert ist und wenigstens eine frei zugängliche, von dem biokompatiblen, elektrisch isolierenden Trägersubstrat mittel- oder unmittelbar umfasste Elektrodenoberfläche aufweist.

### Stand der Technik

Gattungsgemäße implantierbare elektrische Kontaktanordnung, dienen zur elektrischen Signalapplikation und/oder zum elektrischen Signalabgriff an intrakorporalen Gewebeoberflächen, so insbesondere an Oberflächen von Gefäßen, Muskel- oder Nervensträngen. Eine besondere Herausforderung bei der intrakorporalen Verortung derartiger implantierbarer elektrischer Kontaktanordnungen besteht in einer schonenden und möglichst ortsunveränderlichen Anbringung der Kontaktanordnung an einer intrakorporalen Oberfläche ohne diese nachhaltig zu beeinträchtigen bzw. zu irritieren. Dies wirft jedoch hohe Anforderungen an die implantatseitige Fixierung der elektrischen Kontaktanordnung an der jeweils betreffenden Gewebeoberfläche auf, zumal die Gewebeoberfläche durch intrakorporale Temperatur- und Druckänderungen Bewegungen und Formänderungen vollzieht, die durch das medizinische Implantat nicht oder möglichst nicht beeinträchtigt werden sollen. Zudem gilt es den lokalen Flächenkontakt zwischen der Elektrodenoberfläche eines implantatseitig angebrachten Elektrodenkörpers mit einem möglichst unveränderlichen, definierten Flächenkontaktdruck auszubilden, um möglichst gleichbleibende, elektrische Übertragungseigenschaften zu gewährleisten.

Neben einer Vielzahl unterschiedlicher Ausbildungsformen von implantierbaren Elektrodenanordnungen vermögen extraneuronale, manschettenartig ausgebildete, so genannte Cuff-Elektroden, die zirkulär um einen Nervenstrang angelegt werden, elektroneuronale Stimulationen vorzunehmen sowie auch elektroneuronale Signale längs eines Nervenstranges lokal abzugreifen. Durch die zirkular offen ausgebildete Umwicklung der Manschettenelektrode vermag sich diese radial von außen an den Nervenfaserstrang anzuschmiegen und zugleich radialen Formänderungen des Nervenstranges zu folgen.

Die mit dem Nervenstrang in Kontakt tretenden Elektrodenkörper einer als Cuff-Elektrode ausgebildeten implantierbaren, elektrische, Kontaktanordnung sind auf einem biokompatiblen, elektrisch isolierenden, folienartig ausgebildeten Trägersubstrat aufgebracht, das aus einem ebenflächigen Formzustand durch Flächenwicklung und/oder Flächenkrümmung die Gestalt eines geraden Hohlzylinders anzunehmen in der Lage ist. In diesem Zustand der Cuff-Elektrode befinden sich die frei zugänglichen Elektrodenoberflächen der Elektrodenkörper an der Mantelinnenseite des zu einem Hohlzylinder geformten Trägersubstrates und kommen im implantierten Zustand unmittelbar am Epineurium des Nervenstranges zu liegen.

Eine bekannte Cuff-Elektrode ist der Druckschrift EP 0 843 574 B1 zu entnehmen, deren folienartig ausgebildetes Trägersubstrat in Form einer interdigitalen Fingerstruktur ausgebildet ist. Die flexiblen Fingerabschnitte formen durch Folienkrümmung die Gestalt eines geraden Hohlzylinders, dessen Zylinderdurchmesser durch die jeweils frei endenden und wechselweise ineinander greifenden Fingerabschnitte nachgiebig variabel ist. Zur Realisierung und Formstützung der Hohlzylinderform sieht das folienartige Trägersubstrat wenigstens eine Schicht aus Formgedächtnismaterial oder eine unter mechanischer Vorspannung stehenden Materialschicht vor.

Eine alternative Ausgestaltung zur Realisierung einer implantierbaren Cuff-Elektrodenanordnung ist in der Druckschrift EP 3 204 105 B1 beschrieben. In diesem Fall besteht die als Wickelelektrode ausgebildete Manschettenelektrode aus einem folienartigen flexiblen, biokompatiblen Trägersubstrat, vorzugsweise in Form einer Polyimidfolie, an deren einen Trägersubstratoberseite eine aus einer Vielzahl einzelner Elektrodenkörper zusammengesetzte Elektrodenanordnung aufgebracht ist. Die einzelnen Elektrodenkörper der Elektrodenanordnung gelangen in unmittelbaren Oberflächenkontakt mit dem Epineurium des Nervenfaserbündels, zumal sich das Trägersubstrat durch eine entsprechende Einprägung einer mechanischen Folienvorspannung selbständig zu einer geradzylinderförmigen Wickelform aufrollt. Durch die einseitig offen endende Wickeung bilden sich zumindest bereichsweise lose aufeinander gleitende Wickellagen des folienartigen Trägersubstrates aus, durch die eine individuelle Durchmesservariation der hohlzylinderartig um den Nervenstrang anliegenden Cuff-Elektrodenanordnung möglich wird.

Der Anpressdruck, mit dem die Elektrodenoberflächen jeweils an das Epineurium des Nervenfaserbündels anliegen, bestimmt sich im Wesentlichen durch die elastischen Materialeigenschaften des gesamten folienartigen Trägersubstrates.

Der Druckschrift DE 20 2010 015 346 U1 sind Elektrodenanordnungen zu entnehmen, in denen jeweils unter den Elektroden innerhalb eines strangförmigen Substrates ein das gesamte Substrat durchragendes, einstückiges Nitinolband zu formgebenden Zwecken angebracht ist. Das Nitinolband verfügt über ein E-Modul, das sich von dem E-Modul des Trägersubstrats unterscheidet und eine die Form der Elektrodenanordnung, in Art eines Rückrades, stützende Funktion hat.

Die Druckschrift US 2008/0004673 A1 offenbart eine elastisch verformbare Elektrodenmanschette, in deren Manschettenmaterial zu formgebenden Zwecken beispielsweise Formgedächtnismaterial, wie beispielsweise Nitinol eingearbeitet ist.

Die Druckschrift US 2017/087350 A1 offenbart eine Vorrichtung zur Steuerung der Migration von Zellen, z. B. Monozyten, die ein Substrat mit biokompatiblen Elektroden umfasst, die in der Lage sind, ein schwaches elektrisches Feld oder einen schwachen Mikrostrom zu erzeugen.

### Darstellung der Erfindung

Der Erfindung liegt die Aufgabe zugrunde, eine implantierbare elektrische Kontaktanordnung, die wenigstens eine Elektrodenkörperanordnung aufweist, die ansonsten vollständig in ein aus biokompatiblem, elektrisch isolierenden Material gefertigtes Trägersubstrat integriert ist und wenigstens eine frei zugängliche, von den biokompatiblen, elektrisch isolierenden Trägersubstrat mittel- oder unmittelbar umfasste Elektrodenoberfläche aufweist, derart weiterzubilden, so dass der Anpressdruck, mit dem die wenigstens eine Elektrodenoberfläche auf einer intrakorporalen Gewebeoberfläche anliegt, individuell anpassbar ist, ohne dabei das Trägersubstrat in Bezug auf dessen räumliche Dimensionen, insbesondere in Bezug auf die Trägersubstratdicke, zu verändern bzw. wesentlich zu verändern. So soll es möglich sein, den Anpressdruck in der Fläche, längs der das Trägersubstrat auf eine intrakorporale Gewebeoberfläche anliegt, individuell zu designen, d.h. ortsabhängig längs der kontaktierenden Trägersubstratoberfläche einzustellen.

Die Lösung der der Erfindung zugrundeliegende Aufgabe ist im Anspruch 1 angegeben. Vorteilhafte Weiterbildungen des Erfindungsgedankens sind Gegenstand der Unteransprüche sowie der weiteren Beschreibung, insbesondere unter Bezugnahme auf die illustrierten Ausführungsbeispiele zu entnehmen.

In Abkehr von einer naheliegenden Maßnahme der Variation, vor allem der Vergrößerung der Materialdicke des folienartig ausgebildeten Trägersubstrates, um eine lokale größere Materialsteifigkeit und eine damit verbundene Erhöhung der Flächenkontaktkräfte zu erwirken, zeichnet sich die lösungsgemäße implantierbare elektrische Kontaktanordnung dadurch aus, dass innerhalb eines die wenigstens eine Elektrodenkörperanordnung nicht enthaltenen Halbraumes das Trägersubstrat wenigstens einen Raum mittel- oder unmittelbar vollständig umfasst, in dem wenigstens ein Stoff mit einem E-Modul enthalten ist, der sich von einem dem Material des Trägersubstrates zugeordneten E-Modul unterscheidet.

Die lösungsgemäße Maßnahme ermöglicht grundsätzlich eine individuelle Einstellung bzw. Vorgabe der Flächenelastizität bzw. Flächensteifigkeit des folienartig ausgebildeten Trägersubstrates ohne die Dicke des folienartigen Trägersubstrates zu verändern oder wesentlich zu verändern. Insbesondere sind auf diese Weise implantierbare elektrische Kontaktanordnungen realisierbar, deren Elektrodenoberflächen mit einer gegenüber dem übrigen folienartig ausgebildeten Trägersubstrat höheren Anpresskraft auf eine intrakorporale Gewebeoberfläche angedrückt werden.

Auf diese Weise wird einerseits sichergestellt, dass die elektrische Kontaktierung der dauerhaft, stabil und mit gleichbleibenden elektrischen Eigenschaften erfolgt, wohingegen das folienartige Trägersubstrat im übrigen Bereich zum Zwecke der Fixierung mit einer ausreichend geringeren Anpresskraft gewebeschonend am Nervenstrang anliegt. Zu diesem Zweck wird zur Füllung des Raumes wenigstens ein Stoff mit einem höheren E-Modul gewählt im Vergleich zum E-Modul des Materials aus dem das Trägersubstrat gefertigt ist. Auf entsprechende Weise lässt sich die Flächensteifigkeit in Bereichen des Trägersubstrates lokal reduzieren, indem zur Füllung des wenigstens einen Raumes wenigstens ein Stoff mit einem geringeren E-Modul gewählt wird im Vergleich zum E-Modul des Materials aus dem das Trägersubstrat gefertigt ist. Je nach Stoffwahl sowie Vorsehen von Räumen innerhalb des folienartig ausgebildeten Trägersubstrates lassen sich beliebige Steifigkeitsgradienten innerhalb des Trägersubstrates realisieren.

In einer bevorzugten Ausführungsform verfügt das folienartig ausgebildete Trägersubstrat über eine neutrale Faser, die einen die wenigstens Elektrodenkörperanordnung enthaltenen Halbraum des Trägersubstrates gegenüber den wenigstens einen Raum enthaltenen Halbraum des Trägersubstrates trennt.

Der wenigstens eine Raum ist vollständig von dem biokompatiblen, elektrisch isolierenden Material des Trägersubstrates umfasst und ist bezüglich seiner in Folienlängserstreckung orientierten Dimensionierung sehr viel kleiner dimensioniert als das folienartig ausgebildete Trägersubstrat selbst. Dies eröffnet einen problemlosen intrakorporalen Einsatz der lösungsgemäßen implantierbaren elektrischen Kontaktanordnung, zumal der wenigstens eine in dem Trägersubstrat implementierte Stoff keinen Kontakt zum intrakorporalen Milieu besitzt.

Vorzugsweise überlappt der wenigstens eine Raum in orthogonaler Projektion auf die Elektrodenoberfläche zumindest teilweise, vorzugsweise vollständig mit der Elektrodenoberfläche. Auf diese Weise ist sichergestellt, dass zumindest der die Elektrodenoberfläche enthaltende Trägersubstratbereich mit einer erhöhten bzw. individuell eingestellten Anpresskraft lokal gegen eine intrakorporale Gewebeoberfläche gedrückt wird.

Typischerweise besteht das folienartig ausgebildete Trägersubstrat aus einem Polymer, wie beispielsweise Polymid, Liquid Crystal Polymer (LCP), Parylen oder PDMS. Typischerweise verfügen derartige Materialien über ein E-Modul zwischen 1 bis 2 GPa. Bei Verwendung eines metallischen Stoffes zum Ausfüllen des wenigstens einen Raumes können signifikante Steifigkeitsgradienten innerhalb des folienartig ausgebildeten Trägersubstrates eingebracht werden. Die nachfolgende Tabelle zeigt die zu den einzelnen metallischen Werkstoffen zugehörigen E-Module:

| **Material** | **E-Modul in GPa** |
|---|---|
| Gold | 78 |
| Kupfer | 100 bis 130 |
| Aluminium | 70 |
| Nickel | 195 bis 205 |
| Keramik | 160 bis 440 |
| Graphen | ca. 1000 |

Neben den vorstehend, nicht abschließend tabellarisch genannten festen Stoffen, die allesamt zu einer lokalen Versteifung des aus Polymer bestehenden Trägersubstrates beitragen, sind jedoch auch Stoffe mit einem gegenüber dem E-Modul des Trägersubstrates geringeren E-Modul denkbar, die in den wenigstens einen Raum innerhalb des Trägersubstrates eingefügt bzw. eingeschlossen werden können. Beispielsweise eignen sich hierzu Gele, Flüssigkeiten oder auch Gase, die innerhalb des Trägersubstrates einbringbar sind. Bei Verwendung von Gasen oder Flüssigkeiten lässt sich zudem deren zuordenbares E-Modul vom Fülldruck mitbestimmen, mit dem der jeweils gasförmige oder flüssige Stoff den wenigstens einen Raumes innerhalb des folienartig ausgebildeten Trägersubstrates befüllt.

In einer weiteren bevorzugten Ausführungsform sind innerhalb des wenigstens einen Raumes wenigstens zwei Stoffe mit unterschiedlichen E-Modulen eingebracht, vorzugsweise in Form einer schichtförmigen Abfolge zweier Feststoffe, vorzugsweise in Form zweier unterschiedlicher Metallschichten. Durch die Kombination mehrerer Stoffe mit jeweils voneinander abweichenden E-Modulen sind graduelle E-Modul-Profile innerhalb des Trägermodules weitgehend beliebig einstellbar möglich.

In einer weiteren Ausführungsform, vorzugsweise zur lokalen Versteifung des Trägersubstrates wird als wenigstens den einen Raum ausfüllender Stoff ein Wandlerwerkstoff verwendet, der zum Zwecke seiner Form- und/oder Elastizitätsänderung durch eine externe Wirkung, bspw. durch ein externes Energiefeld, änderbar ist. Als geeignete Wandlerwerkstoffe dienen Werkstoffe, wie beispielsweise Bimetalle, Formgedächtnislegierungen, Piezo-Keramiken, elektrostriktive Keramiken, magnetostriktive Legierungen, elektro- oder magnetorheologische Fluide oder ähnliche.

Die implantierbare elektrische Kontaktanordnung weist typischerweise ein flächiges Trägersubstrat auf, das in Form und Größe zur Applikation an einen intrakorporalen Gewebe- oder Nervenstrangbereich geeignet dimensioniert ausgebildet ist. Typische Flächengrößen für das Trägersubstrat, das je nach Anwendung ein- oder mehrfach überdeckend gefaltet und/oder gewickelt sein kann, betragen zwischen einigen mm² und einigen cm², bspw. zwischen 4 mm² und 20 cm², vorzugsweise von 10 mm² bis 6 cm². Das zumeist folienartig ausgebildete Trägersubstrat weist eine Substratdicke von einigen wenigen µm bis einige hundert µm auf, bspw. von 2 µm bis 650 µm, vorzugsweise 10 µm bis 100 µm.

Der wenigstens eine von dem Trägersubstratmaterial vollständig, d.h. hermetisch, umschlossene Raum, der vorzugsweise eine kubische oder quaderförmige Raumform besitzt, ist sehr viel kleiner dimensioniert als die Dimension des Trägersubstrat in seiner Längserstreckung. Maximal umfasst der wenigstens eine Raum ein Volumen von 17 mm³. Der mit einem Stoff befüllte Raum, dessen E-Modul sich vom E-Modul des Trägersubstrates unterscheidet, vermag die mechanischen Eigenschaften des Trägersubstrats lokal nur im Bereich um den Raum zu beeinflussen. Aufgrund der kleinen Dimensionierung des wenigstens einen Raumes im Verhältnis zur Größe des Trägersubstrates übt der in dem Raum enthaltene Stoff keine die gesamte Raumform des Trägersubstrates beeinflussende Stützkräfte aus. Auch im Falle von einer Vielzahl innerhalb des Trägersubstrats verteilt angeordneter und mit wenigstens einem Stoff befüllte Räume, die jeweils inselartig beabstandet zueinander angeordnet sind wirken die Raumbereiche mit den jeweils zum E-Modul des Trägersubstrates unterschiedlichen E-Module bereichsweise versteifend oder erweichend, jedoch nicht die Raumform des Trägersubstrates formprägend, da die Räume keine zusammenhängende Stützwirkung zu entfalten vermögen.

So sieht ein Ausführungsbeispiel eine erste Vielzahl von jeweils mit wenigstens einem Stoff befüllten Räumen längs einer ersten Geraden oder längs einer ersten Ebene arrayförmig jeweils voneinander beabstandet innerhalb des Trägersubstrates verteilt vor.

In einem weiteren Ausführungsbeispiel ist zusätzlich zu der vorstehenden ersten Vielzahl eine zweite Vielzahl von jeweils mit wenigstens einem Stoff befüllten Räumen jeweils beabstandet zueinander längs einer zweiten Geraden oder längs einer zweiten Ebene innerhalb des Trägersubstrates verteilt angeordnet. Vorzugsweise sind die erste und zweite Gerade oder die erste und zweite Ebene jeweils parallel zueinander orientiert.

Ergänzend zu der ersten und zweiten Vielzahl an jeweils mit wenigstens einem Stoff befüllten Räumen sieht ein weitere Ausführungsbeispiel eine weitere Vielzahl von jeweils mit wenigstens einem Stoff befüllten Räumen jeweils beabstandet zueinander längs einer weiterenn Geraden oder längs einer weiteren Ebene innerhalb des Trägersubstrates verteilt vor.

Mit einer Vielzahl derartiger mit einem Stoff befüllten Räumen sind folienartig ausgebildete Trägersubstratbereiche mit individuell eingeprägten Festigkeiten und/oder Steifigkeiten vorgebbar.

Insbesondere im Falle einer um einen Nervenstrang umschlingbaren Cuff-Elektrodenanordnung lässt sich der unmittelbar am Nervenstrang anliegende Trägersubstratbereich und/oder die Nervenstrang nahen Umschlingungen des Trägersubstrates mit einer möglichst geringen Flächensteifigkeit ausbilden, indem in diesem Flächenbereich des Trägersubstrats keine derartigen mit einem Stoff befüllten Räume eingebracht sind oder lediglich mit einem Stoff befüllte Räume eingebracht sind, dessen E-Modul signifikant kleiner als das E-Modul des Trägersubstratmaterial gewählt ist. In dem radial daran anschliessenden Trägersubstratbereich der Cuff-Elektrodenanordnung sind mit Stoff befüllte Räume eingebracht, dessen E-Modul größer gewählt ist als das E-Modul des Trägersubstratmaterials. Auf diese Weise kann ein von radial Aussen wirkender erhöhter Anpressdruck auf die innenliegenden Trägersubstratlagen ausgeübt werden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:

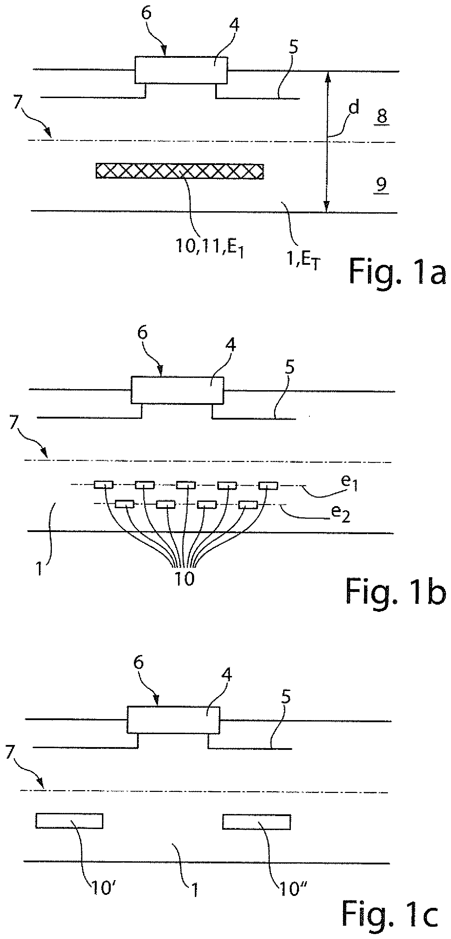

- Fig. 1 a, b, c: Längsschnittdarstellung durch jeweils ein Trägersubstrat mit Elektrodenanordnung und wenigstens Raum,

### Wege zur Ausführung der Erfindung, gewerbliche Verwendbarkeit

Fig. 1a zeigt einen Längsschnitt durch ein folienartig ausgebildetes Trägersubstrat 1, vorzugsweise in Form einer Polyimid-Folie, mit einer Trägersubstratoberseite 2 und einer dieser gegenüberliegenden Trägersubstratunterseite 3. Innerhalb des Trägersubstrats 1 ist eine Elektrodenanordnung eingebettet, die über wenigstens einen Elektrodenkörper 4 verfügt, der über innerhalb des Trägersubstrats 1 verlaufende elektrische Zu- und Ableitungen 5 kontaktiert ist. Der Elektrodenkörper 4 verfügt über eine frei zugängliche Elektrodenoberfläche 6. Nicht notwendigerweise, jedoch in vorteilhafter Form überragt der Elektrodenkörper 4 die Trägersubstratoberseite 2, die im implantierten Zustand einer nicht weiter dargestellten intrakorporalen Gewebeoberfläche zugewandt orientiert ist, so dass die Elektrodenoberfläche 6 in Flächenkontakt mit der Gewebeoberfläche tritt.

Das folienartig ausgebildete Trägersubstrat 1 weist eine neutrale Faser 7 auf, die das Trägersubstrat 1 in einen oberen Halbraum 8 und einen unteren Halbraum 9 teilt. Der obere Halbraum 8 umfasst die Elektrodenanordnung, wohingegen der untere Halbraum des Trägersubstrates 1 wenigstens einen Raum 10 umfasst, der vom Material des Trägersubstrates 1 vollständig eingeschlossen ist und in dem wenigstens ein Stoff 11 enthalten ist, der über ein Elastizitätsmodul E1 verfügt, das sich von dem Elastizitätsmodul Et des Trägersubstrats 1 unterscheidet.

In dem in Fig. 1a dargestellten Ausführungsbeispiel ist der Raum 10 in orthogonaler Projektion auf die Trägersubstratoberseite 2 vollständig überlappend unterhalb des Elektrodenkörpers 4 lokal angeordnet.

Eine bevorzugte Stoffwahl betrifft die Verwendung eines metallischen Stoffes 11 innerhalb des Raums 10, vorzugsweise in Form einer einstückigen metallischen Schicht, die von dem biokompatiblen, elektrisch isolierenden Material des Trägersubstrats 1 allumfassend umschlossen ist. Der metallische Stoff 11 vermag das Trägersubstrat 1 lokal zu versteifen, wodurch in Ausbildung des Trägersubstrates als Cuff-Elektrodenanordnung eine lokale Erhöhung des Anpressdruckes im Bereich des Elektrodenkörpers 4 an eine intrakorporale Gewebeoberfläche, vorzugsweise in Form einer Nervenstrangoberfläche, erzielt wird. Die zusätzliche Implementierung bzw. Integration eines Stoffes 11 innerhalb des Raumes 10 ist nicht mit einer Vergrößerung der Trägersubstratdicke d verbunden.

Die Anordnung, Dimensionierung und räumliche Ausbildung des Raumes 10 innerhalb eines Trägersubstrates können vielgestaltig gewählt werden.

Figur 1b illustriert ein alternatives Ausführungsbeispiel, das eine Vielzahl einzelner Räume 10' innerhalb des Trägersubstrates 1 vorsieht, die allesamt einheitlich mit einem Stoff 11 oder mit jeweils unterschiedlichen Stoffen, je nach gewünschter Einstellung der Steifigkeit des Trägersubstrats 1 im Bereich wenigstens eines Elektrodenkörpers 4 befüllt sind. In Fig. 1b ist die Vielzahl der Räume 10' längs einer parallel zur neutralen Faser 7 verlaufenden Ebene e1 angeordnet. Denkbar sind auch alternative Anordnungsmuster der Vielzahl einzelner Räume 10', beispielsweise innerhalb zweier oder mehr parallel zueinander angeordneter Ebenen e1, e2 etc.

Fig. 1c illustriert ein weiteres Ausführungsbeispiel mit wenigstens zwei Räumen 10", die jeweils in orthogonaler Projektion zur Trägersubstratoberseite 2 lateral neben dem Elektrodenkörper 4 angebracht sind. In diesem Fall weist das Trägersubstrat 1 bei entsprechender Befüllung der Räume 10' mit einem Stoff, der über ein höheres E-Modul verfügt als das E-Modul des Trägersubstrates 1, im Bereich der Elektrodenanordung eine geringere Steifigkeit als in den seitlich daran angrenzenden Trägersubstratbereichen auf.

Allen denkbaren Ausführungsbeispielen ist gemein, dass durch Vorsehen eines Raumes oder einer Vielzahl jeweils mit wenigstens einem Stoff befüllten Räumen innerhalb des Trägersubstrates 1 ohne Dickenzunahme des folienartig ausgebildeten Trägersubstrates beliebig konfektionierbare Steifigkeitsgradienten innerhalb des Trägersubstrats 1 eingebracht werden können. Nicht zuletzt bedingt durch die freie Stoffwahl zur Befüllung der einzelnen Räume, die neben Festkörpern auch gasförmige oder insbesondere flüssige bzw. gelartige Stoffe umfassen können, kann das Flächensteifigkeitsverhalten des gesamten Trägersubstrates individuell und fein dosiert konfektioniert werden.

Auch ist der Einsatz von gewebe- oder faserartigen Stoffen denkbar, die einzeln oder lagenweise lokal begrenzt innerhalb des Trägersubstrates integrierbar sind. Beispielsweise im Falle einer Gewebelage kann diese matrixartig von dem biokompatiblen, elektrisch nicht leitenden Material des Trägersubstrates umfasst sein.

Das lösungsgemäße Vorsehen wenigstens eines Raumes innerhalb des Trägersubstrates, der von einem Stoff befüllt ist, dessen E-Modul sich von dem E-Modul des Materials des Trägersubstrates unterscheidet, dient auch zur Kompensation von innerhalb des Trägersubstrates auftretenden mechanischen Spannungen, die durch die Integration der Elektrodenanordnung innerhalb des Trägersubstrates bedingt sind. Derartige mechanische Spannungen können zu überhöhten Materialbeanspruchungen innerhalb des Trägersubstrates führen und letztlich die Lebensdauer der implantierbaren elektrischen Kontaktanordnung begrenzen. Insbesondere die Ausgestaltung der implantierbaren elektrischen Kontaktanordnung in Art einer an sich bekannten Cuff-Elektrode führt zu materialintrinsischen mechanischen Spannungen, die mit Hilfe wenigstens eines mit einem Stoff befüllten Raumes innerhalb des Trägersubstrates vollständig oder zumindest weitgehend kompensiert werden können.

### Bezugszeichenliste

- 1: Trägersubstrat
- 2: Trägersubstratoberseite
- 3: Trägersubstratunterseite
- 4: Elektrodenkörper
- 5: elektr. Zu- und Ableitung
- 6: Elektrodenoberfläche
- 7: neutrale Faser
- 8: die Elektrodenkörperanordnung enthaltender Halbraum
- 9: den wenigstens einen Raum enthaltenden Halbraum
- 10, 10', 10": Raum
- 11: Stoff
- e1, e2: Anordnungsebene für die Räume
- d: Foliendicke des Trägersubstrats
- E1: E-Modul des Stoffes
- E_{T}: E-Modul des Trägersubstrates

## Patentansprüche

1. Implantierbare elektrische Kontaktanordnung, die wenigstens eine Elektrodenkörperanordnung (4) aufweist, die ansonsten vollständig in ein aus biokompatiblem, elektrisch isolierendem Material gefertigtes Trägersubstrat (1) integriert ist und wenigstens eine frei zugängliche, von dem biokompatiblen, elektrisch isolierenden Trägersubstrat (1) mittel- oder unmittelbar umfasste Elektrodenoberfläche (6) aufweist,
**dadurch gekennzeichnet, dass** das Trägersubstrat (1) folienartig ausgebildet ist, mit einer die wenigstens eine frei zugängliche Elektrodenoberfläche (6) aufweisenden Trägersubstratoberseite (2), einer dieser gegenüberliegenden Trägersubstratunterseite (3) sowie einer Folienlängserstreckung,
dass innerhalb eines die wenigstens eine Elektrodenkörperanordnung (4) nicht enthaltenden Halbraumes das Trägersubstrat (1) wenigstens einen Raum (10) vollständig umfasst, in dem wenigstens ein Stoff (11) mit einem E-Modul enthalten ist, der sich von einem dem Material des Trägersubstrats (1) zugeordneten E-Modul unterscheidet, und
dass der wenigstens eine Raum (10), in dem der wenigstens eine Stoff (11) enthalten ist, eine parallel zur Trägersubstratober- (2) und -unterseite (3) orientierte Erstreckung besitzt, die kleiner als die Folienlängserstreckung dimensioniert ist.

2. Implantierbare elektrische Kontaktanordnung nach Anspruch 1,
**dadurch gekennzeichnet, dass** dem Trägersubstrat (1) mit der wenigstens einen Elektrodenkörperanordnung (4) eine neutrale Faser (7) zugeordnet ist, die den die wenigstens eine Elektrodenkörperanordnung (4) enthaltenden Halbraum (8) gegenüber dem den wenigstens einen Raum (10) enthaltenden Halbraum trennt (9).

3. Implantierbare elektrische Kontaktanordnung nach Anspruch 1 oder 2,
**dadurch gekennzeichnet, dass** der wenigstens eine Raum (10) in orthogonaler Projektion zur Elektrodenoberfläche (6) zumindest teilweise mit der Elektrodenoberfläche (6) überlappt.

4. Implantierbare elektrische Kontaktanordnung nach einem der Ansprüche 1 bis 3,
**dadurch gekennzeichnet, dass** das Trägersubstrat (1) aus einem Polymer
eines der nachstehenden Polymere besteht: Polyimid, Liquid crystal polymer (LCP), Parylen oder PDMS.

5. Implantierbare elektrische Kontaktanordnung nach einem der Ansprüche 1 bis Anspruch 4,
**dadurch gekennzeichnet, dass** der Stoff (11) gasförmig, flüssig oder fest ist.

6. Implantierbare elektrische Kontaktanordnung nach einem der Ansprüche 1 bis Anspruch 5,
**dadurch gekennzeichnet, dass** wenigstens zwei Stoffe mit unterschiedlichen E-Modulen innerhalb des wenigstens eines Raumes (10) enthalten sind.

7. Implantierbare elektrische Kontaktanordnung nach einem der Ansprüche 1 bis 6,
**dadurch gekennzeichnet, dass** der wenigstens eine Stoff (11) ein Wandlerwerkstoff ist.

8. Implantierbare elektrische Kontaktanordnung nach Anspruch 7,
**dadurch gekennzeichnet, dass** der Wandlerwerkstoff ein Werkstoff aus der nachfolgenden Stoffgruppe ist: Bimetall, Formgedächtnislegierung, Piezo-Keramik, elektrostriktive Keramik, magnetostriktive Legierung, elektro- oder magnetorheologisches Fluid.

9. Implantierbare elektrische Kontaktanordnung nach einem der Ansprüche 1 bis 8,
**dadurch gekennzeichnet, dass** der wenigstens eine Raum (10) ein Raumvolumen von maximal 17 mm³ einschließt.

10. Implantierbare elektrische Kontaktanordnung nach einem der Ansprüche 1 bis 9,
**dadurch gekennzeichnet, dass** der Raum (10) bezüglich seiner in Folienlängserstreckung orientierten Dimension um wenigstens eine Größenordnung kleiner dimensioniert ist.

11. Implantierbare elektrische Kontaktanordnung nach einem der Ansprüche 1 bis 10,
**dadurch gekennzeichnet, dass** der wenigstens eine Raum (10) und der in dem wenigstens einen Raum (10) enthaltene Stoff (11) derart dimensioniert und gewählt sind, dass der wenigstens eine Raum (10) und der in dem wenigstens einen Raum (10) enthaltene Stoff (11) keine die Raumform der implantierbaren elektrischen Kontaktanordnung bestimmende Formkraft auszuüben vermag.

12. Implantierbare elektrische Kontaktanordnung nach einem der Ansprüche 1 bis 11,
**dadurch gekennzeichnet, dass** eine erste Vielzahl von jeweils mit wenigstens einem Stoff (11) befüllten Räumen (10) längs einer ersten Geraden oder längs einer ersten Ebene (e1) arrayförmig jeweils voneinander beabstandet innerhalb des Trägersubstrates (1) verteilt angeordnet ist.

13. Implantierbare elektrische Kontaktanordnung nach Anspruch 12,
**dadurch gekennzeichnet, dass** eine zweite Vielzahl von jeweils mit wenigstens einem Stoff (11) befüllten Räumen (10) jeweils beabstandet zueinander längs einer zweiten Geraden oder längs einer zweiten Ebene (e2) innerhalb des Trägersubstrates (1) verteilt angeordnet ist.

14. Implantierbare elektrische Kontaktanordnung nach Anspruch 13
**dadurch gekennzeichnet, dass** die erste und zweite Gerade oder die erste und zweite Ebene (e1, e2) jeweils parallel zueinander orientiert sind.

15. Implantierbare elektrische Kontaktanordnung nach Anspruch 13 oder 14, **dadurch gekennzeichnet, dass** wenigstens eine weitere Vielzahl von jeweils mit wenigstens einem Stoff (11) befüllten Räumen (10) jeweils beabstandet zueinander längs einer weiteren Geraden oder längs einer weiteren Ebene innerhalb des Trägersubstrates (1) verteilt angeordnet ist.

## Claims

1. An implantable electrical contact arrangement, which comprises at least one electrode element arrangement (4), which is otherwise completely integrated into a carrier substrate (1) made of a biocompatible, electrically insulating material, and comprises at least one freely-accessible electrode surface (6) directly or indirectly surrounded by the biocompatible electrically insulating carrier substrate (1),
**characterised in that** the carrier substrate (1) is filmlike in design with a carrier substrate upper side (2) comprising the at least one freely accessible electrode surface (6), a carrier substrate lower side (3) opposite this as well as a longitudinal extent of the foil,
**in that** within a half space not containing the at least one electrode element arrangement (4), the carrier substrate (1) completely surrounds at least one space (10) in which at least one material (11) with an E-module is contained, which differs from the E-module assigned to the material of the carrier substrate (1), and
**in that** the at least one space (10), in which the at least one material (11) is contained, has an extent orientated in parallel to the carrier substrate upper side (2) and lower side (3), which is smaller in dimension than the longitudinal extent of the foil.

2. The implantable electrical contact arrangement according to claim 1,
**characterised in that** assigned to the carrier substrate (1) with the at least one electrode element arrangement (4) is a neutral fibre (7) which separates the half space (8) containing the at least one electrode element arrangement (4) from the half space (9) containing the at least one space (10).

3. The implantable electrical contact arrangement according to claims 1 or 2,
**characterised in that** in orthogonal projection to the electrode surface (6) the at least one space (10) at least partially overlaps with the electrode surface (6) .

4. The implantable electrical contact arrangement according to any one of claims 1 to 3,
**characterised in that** the carrier substrate (1) is made of one of the following polymers: polyimide, liquid crystal polymer (LCP), parylene or PDMS.

5. The implantable electrical contact arrangement according to any one of claims 1 to 4,
**characterised in that** the material (11) is gaseous, fluid or solid.

6. The implantable electrical contact arrangement according to any one of claims 1 to 5,
**characterised in that** at least two materials with different E-modules are contained within the at least one space (10).

7. The implantable electrical contact arrangement according to any one of claims 1 to 6,
**characterised in that** the at least one material (11) is a transducer material.

8. The implantable electrical contact arrangement according to claim 7,
**characterised in that** the transducer material is from the following group: bi-metal, shape-memory alloy, piezo-ceramic, electrostrictive ceramic, magnetostrictive alloy, electro or magnetorheological fluid.

9. The implantable electrical contact arrangement according to any one of claims 1 to 8,
**characterised in that** the at least one space (10) encloses a spatial volume of a maximum of 17 mm³.

10. The implantable electrical contact arrangement according to any one of claims 1 to 9,
**characterised in that** in relation to its dimension orientated in the longitudinal extent of the film, the space (10) is dimensioned one order of magnitude smaller.

11. The implantable electrical contact arrangement according to any one of claims 1 to 10,
**characterised in that** the at least one space (10) and the material (11) contained in the at least one space (10) are dimensioned and selected in such at way that the at least one space (10) and the material (11) contained in the at least one space (10) cannot exert any shaping force determining the spatial shape of the implantable electrical contact arrangement.

12. The implantable electrical contact arrangement according to any one of claims 1 to 11,
**characterised in that** a first plurality of spaces (10) each filled with at least one material (11) is arranged along a first straight line or along a first plane (e1), distributed in an array form each at a distance from one another within the carrier substrate (1).

13. The implantable electrical contact arrangement according to claim 12,
**characterised in that** a second plurality of spaces (10) each filled with at least one material (11) is arranged along a second straight line or along a second plane (e2) distributed each at a distance from one another within the carrier substrate (1).

14. The implantable electrical contact arrangement according to claims 13,
**characterised in that** the first and the second straight line or the first and the second plane (e1, e2) are each orientated in parallel to each other.

15. The implantable electrical contact arrangement according to claim 13 or 14,
**characterised in that** at least one further plurality of spaces (10) each filled with at least one material (11) is arranged along a further straight line or along a further plane distributed each at a distance from one another within the carrier substrate (1).

## Revendications

1. Dispositif de contact électrique implantable, qui comporte au moins un dispositif de corps d'électrode (4), qui est intégré autrement complètement dans un substrat porteur (1) fabriqué à partir d'un matériau biocompatible, électro-isolant et comporte au moins une surface d'électrode (6) librement accessible, comprise directement ou indirectement par le substrat porteur (1) biocompatible, électro-isolant,
**caractérisé en ce que** le substrat porteur (1) est constitué sous forme de feuille, avec un côté supérieur de substrat porteur (2) comportant au moins une surface d'électrode (6) librement accessible, un côté inférieur de substrat porteur (3) opposé à celui-ci ainsi qu'une extension longitudinale de feuille,
**en ce qu'**à l'intérieur d'un demi-espace ne contenant pas au moins un dispositif de corps d'électrode (4), le substrat porteur ((1) comprend complètement au moins u espace (10) dans lequel est contenue au moins une matière (11) avec un module d'élasticité, qui se différencie d'un module d'élasticité attribué au matériau du substrat porteur (1), et
**en ce qu'**au moins un espace (10) dans lequel est contenue au moins une matière (11), possède une extension orientée parallèlement au côté supérieur (2) et au côté inférieur (3) du substrat porteur, qui est dimensionnée plus petite que l'extension longitudinale de la feuille.

2. Dispositif de contact électrique implantable selon la revendication 1,
**caractérisé en ce qu'**une fibre neutre (7) est attribuée au substrat porteur (1) avec au moins un dispositif de corps d'électrode (4), qui sépare le demi-espace (8) contenant au moins un dispositif de corps d'électrode (4) du demi-espace contenant au moins un espace (10).

3. Dispositif de contact électrique implantable selon la revendication 1 ou 2,
**caractérisé en ce qu'**au moins un espace (10) chevauche au moins en partie la surface d'électrode (6) en projection orthogonale par rapport à la surface d'électrode (6).

4. Dispositif de contact électrique implantable selon l'une quelconque des revendications 1 à 3,
**caractérisé en ce que** le substrat porteur (1) est composé d'un polymère d'un des polymères suivants : polyimide, polymère à cristaux liquides (PCL), pyralène ou polydiméthylsiloxane (PDMS).

5. Dispositif de contact électrique implantable selon l'une quelconque des revendications 1 à 4, **caractérisé en ce que** la matière (11) est gazeuse, liquide ou solide.

6. Dispositif de contact électrique implantable selon l'une quelconque des revendications 1 à 5,
**caractérisé en ce qu'**au moins deux matières avec des modules d'élasticité différents sont contenues à l'intérieur d'au moins un espace (10).

7. Dispositif de contact électrique implantable selon l'une quelconque des revendications 1 à 6, **caractérisé en ce qu'**au moins une matière (11) est une matière de transducteur.

8. Dispositif de contact électrique implantable selon la revendication 7,
**caractérisé en ce que** la matière de transducteur est une matière appartenant au groupe de matières suivant : bimétal, alliage à mémoire de forme, piézo-céramique, céramique électrostrictive, alliage magnétostrictif, fluide électro- ou magnétorhéologique.

9. Utilisation selon l'une quelconque des revendications 1 à 8,
**caractérisée en ce qu'**au moins un espace (10) forme un volume d'espace maximal de 17 mm³.

10. Dispositif de contact électrique implantable selon l'une quelconque des revendications 1 à 9,
**caractérisé en ce que** l'espace (10) est dimensionné plus petit d'au moins un ordre de grandeur eu égard à sa dimension orientée dans l'extension longitudinale de la feuille.

11. Dispositif de contact électrique implantable selon l'une quelconque des revendications 1 à 10,
**caractérisé en ce qu'**au moins un espace (10) et la matière (11) contenue dans au moins un espace (10) sont dimensionnés et sélectionnés de telle sorte qu'au moins un espace (10) et la matière (11) contenue dans au moins un espace (10) ne sont capables d'exercer aucune force de formation déterminant la forme d'espace du dispositif de contact électrique implantable.

12. Dispositif de contact électrique implantable selon l'une quelconque des revendications 1 à 11,
**caractérisé en ce que** des espaces (10) remplis respectivement d'au moins une matière (11) sont disposés en première pluralité, répartis le long d'une première droite ou le long d'un premier plan (e1) en forme de réseau, respectivement à distance les uns des autres à l'intérieur du substrat porteur (1).

13. Dispositif de contact électrique implantable selon la revendication 12,
**caractérisé en ce que** des espaces (10) remplis respectivement d'au moins une matière (11) sont disposés en deuxième pluralité, répartis le long d'une deuxième droite ou le long d'un deuxième plan (e2) respectivement à distance les uns des autres à l'intérieur du substrat porteur (1).

14. Dispositif de contact électrique implantable selon la revendication 13,
**caractérisé en ce que** la première et la deuxième droite ou le premier et le deuxième plan (e1, e2) sont orientés respectivement parallèlement l'un à l'autre.

15. Dispositif de contact électrique implantable selon la revendication 13 ou 14,
**caractérisé en ce que** des espaces (10) remplis respectivement d'au moins une matière (11) sont disposés dans une autre pluralité, répartis le long d'une autre droite ou le long d'un autre plan, respectivement à distance les uns des autres à l'intérieur du substrat porteur (1).
